# EUROPEAN PATENT SPECIFICATION

(11) **EP 2 568 934 B1**
(45) Date of publication and mention of the grant of the patent: **06.04.2016**
(21) Application number: 11724783.3
(22) Date of filing: 02.05.2011
(51) Int. Cl.: A61F 5/445

(54) **OSTOMY PORT**
OSTOMIEANSCHLUSS
ORIFICE DE STOMIE

(30) Priority: 10.01.2011 US 431084 P; 14.07.2010 WO PCT/IL2010/000565; 02.05.2010 US 330359 P
(43) Date of publication of application: 20.03.2013
(73) Proprietor: B.Braun Medical SAS, 92100 Boulogne-Billancourt (FR)
(72) Inventor: HANUKA, David, 30095 Ramat-Yishai (IL); OR, Meir, 20196 Doar-Na Misgav (IL)
(74) Representative: Mahler, Peter
(86) International application number: PCT/IB2011/051933
(87) International publication number: WO 2011/138728

(56) References cited:
- WO-A1-90/07311
- WO-A1-96/32904
- WO-A1-99/43277
- WO-A1-2009/155537
- US-A- 5 569 216

## Description

### FIELD AND BACKGROUND OF THE INVENTION

The present invention, relates to the field of prosthetic implants, and more particularly, to an Ostomy port for use following Ostomy cases such as Colostomy, Ileostomy or Urostomy, or for fecal incontinence.

An Ostomy is a surgical procedure wherein an opening (stoma) is created in the body, for example, for the discharge of body wastes. When performing an ostomy, the physician will generally form a stoma in the abdominal wall and attach an end or a side of a healthy portion of the intestine (large or small intestine, depending on the type of ostomy) to the stoma from the visceral side of the abdominal wall or, alternatively, pass the intestinal portion through the stoma and attach it to the outside of the abdominal wall. The stoma may be permanently left in a patient suffering from a condition for when it is no longer possible for the intestinal content to pass out via the anus, for example, due to colon cancer, diverticulitis, trauma, or inflammatory bowel disease, or may be temporary, as may be the case following an operation on a section of the bowel (small intestine and/or large intestine) requiring a healing period.

Following a stoma operation, a stomal insert may be inserted through the stoma into the intestine and may serve as an ostomy port for preventing the body wastes from coming in contact with the external abdominal wall as they are expelled through the stoma. The ostomy port may form part of an ostomy containment system which may serve to control the flow of the body wastes out the stoma.

US 2007/0049878 A1 discloses "a bowel management system includes a waste collection catheter having at least two distinct sections. The first section is patient proximal when disposed in the patient's rectum and has durometer hardness in the range of about 50A to about 90A. The second catheter section is connected to the first section and has durometer hardness in the range of about 5A to about 49A. A selectively collapsible, substantially spherical retention balloon is attached coaxially and exterior of the first catheter section such that the proximal-most end of the retention balloon is coincident to the proximal-most end of the first section of the waste collection catheter, the substantially spherical retention balloon having an inflated size so as to be sufficiently large enough to retain the patient proximal end of the catheter in the patient's rectum without being so large as to trigger a defecatory response in the patient."

US 2005/0054996 A1 discloses "the end of the elongated tubular element of the appliance that is designed to be inserted into a body cavity or vessel is formed entirely of soft, compliant material. That end carries an inflatable balloon formed in its fully inflated shape. The balloon is inflated to a predetermined low pressure level to prevent pressure necrosis in the adjacent tissue. A method and apparatus for introducing the soft end of the appliance into the body cavity are also provided. The introducer apparatus includes rigid core surrounded by a soft, compliant sleeve. The sleeve extends beyond the rigid core to form an invertable section. The soft end of the appliance is situated adjacent the apparatus, the balloon is wrapped around the apparatus, and the sleeve section is inverted over the appliance, compressing the balloon and forming a soft, rounded insertion tip. The unit is then introduced into the body cavity. After the appliance is separated from the apparatus, the apparatus is withdrawn."

US 2009/0247969 A1 discloses "a waste management system includes a waste transport device, a waste collection device, and an insertion device. The waste transport device includes a collection member with a distal end opening having a first cross-sectional area and a proximal end opening having a second cross-sectional area less than the first cross-sectional area, and a retention cuff disposed about an outer surface of the collection member. The insertion device is positioned about the collection member and retention cuff to retain the collection member and retention cuff in a collapsed configuration."

WO 2009/155537 A1 describes "a stoma extender includes a first end for insertion into a stoma for diverting stomal effluent into the stoma extender before the effluent exits the stoma; a second end for remaining external of the stoma, for providing a discharge exit for stomal effluent; and a conduit portion coupled between the first and second ends for communicating stomal effluent through the stoma extender, wherein the length of the conduit portion is adjustable stably, to permit adaptation of the stoma extender to an individual's stoma."

US 5,569,216 describes "a multipurpose colostomy device for fixing in the stoma or rectum of a human body, includes an internal balloon, a ring configured external balloon surrounding the internal balloon, a connecting tube disposed under the both internal and external balloons, a joint tube operatively connected to a drainage hose and disposed under connecting tube, a supporting plate disposed between the connecting and joint tubes for fixing the colostomy to the abdominal wall, and an L-shaped supply tube containing a pair of air passages, a washing fluid passage and an enema fluid passage."

WO 96/32904 describes the problem which is solved by a prosthesis for bowel evacuation control at the incontinence of an artificial or natural anus in accordance with the invention is how to provide simple, safe and reliable control of bowel evacuation in artificial or natural incontinence, where under artificial incontinence colostomy, i.e. a surgically formed opening in the large intestine through the abdominal wall is understood, and under natural incontinence the incontinence of the anal sphincter is understood. The illustrated prosthesis inserted in a colostomy, i.e. a bowel extended through the abdominal wall, consists of an inner ring which continues into a pellicular tube and this into a faceplate, and of a cover. In this embodiment the inner ring is carried out as a ring made of pliable, organism-friendly material with a built-in reinforcement providing a sufficient force for reexpansion of the ring and preventing its deformation when being inserted into the bowel lumen. The inner ring continues along its entire circumference into the tube whose thin walls are made of a material with similar characteristics as the inner ring. The diameter of an unstretched tube is a little smaller than the diameter of the inner ring. The length of the tube in this embodiment is a little smaller than the thickness of the abdominal wall. At the end lying opposite to the inner ring the tube continues into a faceplate which is preferably of circular form and made of firm material or dimensioned so that it is firm yet pliable. The plate has an opening in the centre in which the cover is inserted which prevents the faeces from escaping."

Additional background art includes US 6033390, US 4338937A, US 4534761A, and EP 2027835A1.

### SUMMARY OF THE INVENTION

According to the present invention, there is provided an ostomy port for conducting waste content from an intestinal portion in an abdominal cavity through a stoma in an abdominal wall comprising; a cover for covering the stoma; a fixation element for anchoring the port to a visceral side of the abdominal wall; and a pliable, axially elastic tube interconnecting the cover and the fixation element and sized to apply a tensile force to the cover and to the fixation element. The ostomy port in accordance with the invention is characterized in that at least one of said cover, said fixation element, and said elastic tube is adapted to deform for regulating a pressure applied by said fixation element on said abdominal wall due to said tensile force; and the pressure applied by said fixation element due to said tensile force decreases to less than 10 mmHg when a colonic pressure is equal to or greater than approximately 10 mmHg. proximal end opening having a second cross-sectional area less than the first cross-sectional area, and a retention cuff disposed about an outer surface of the collection member. The insertion device is positioned about the collection member and retention cuff to retain the collection member and retention cuff in a collapsed configuration."

WO 2009/155537 A1, "Ostomy Appliances For Directing Effluent Output", describes "a stoma extender includes a first end for insertion into a stoma for diverting stomal effluent into the stoma extender before the effluent exits the stoma; a second end for remaining external of the stoma, for providing a discharge exit for stomal effluent; and a conduit portion coupled between the first and second ends for communicating stomal effluent through the stoma extender, wherein the length of the conduit portion is adjustable stably, to permit adaptation of the stoma extender to an individual's stoma."

U. S. Patent No. 5,569,216 to Kim describes "a multipurpose colostomy device for fixing in the stoma or rectum of a human body, includes an internal balloon, a ring configured external balloon surrounding the internal balloon, a connecting tube disposed under the both internal and external balloons, a joint tube operatively connected to a drainage hose and disposed under connecting tube, a supporting plate disposed between the connecting and joint tubes for fixing the colostomy to the abdominal wall, and an L-shaped supply tube containing a pair of air passages, a washing fluid passage and an enema fluid passage."

WO 96/32904, "Prosthesis For Bowel Evaluation Control-Colostomy Tube", describes "the problem which is solved by a prosthesis for bowel evacuation control at the incontinence of an artificial or natural anus in accordance with the invention is how to provide simple, safe and reliable control of bowel evacuation in artificial or natural incontinence, where under artificial incontinence colostomy, i.e. a surgically formed opening in the large intestine through the abdominal wall is understood, and under natural incontinence the incontinence of the anal sphincter is understood. The illustrated prosthesis inserted in a colostomy, i.e. a bowel (1) extended through the abdominal wall (2), consists of an inner ring (3) which continues into a pellicular tube (4) and this into a faceplate (5), and of a cover (8). In this embodiment the inner ring (3) is carried out as a ring made of pliable, organism-friendly material with a built-in reinforcement (9) providing a sufficient force for reexpansion of the ring (3) and preventing its deformation when being inserted into the bowel lumen. The inner ring (3) continues along its entire circumference into the tube (4) whose thin walls are made of a material with similar characteristics as the inner ring (3). The diameter of an unstretched tube (4) is a little smaller than the diameter of the inner ring (3). The length of the tube (4) in this embodiment is a little smaller than the thickness of the abdominal wall (2). At the end lying opposite to the inner ring (3) the tube (4) continues into a faceplate (5) which is preferably of circular form and made of firm material or dimensioned so that it is firm yet pliable. The plate (5) has an opening in the centre in which the cover (8) is inserted which prevents the faeces from escaping."

Additional background art includes US Patent 6033390 (to von Dyck), US Patent 4338937A (to Lerman Sheldon), US Patent 4534761A (to Raible Donald), and EP 2027835A1 (to Axelsson et al).

### SUMMARY OF THE INVENTION

According to an aspect of some embodiments of the present invention, there is provided a stomal insert for conducting waste content from an intestinal portion in an abdominal cavity through a stoma in an abdominal wall comprising; a cover for covering the stoma; a fixation element for anchoring the stomal insert to a visceral side of the abdominal wall; and a pliable, axially elastic tube interconnecting the cover and the fixation element and sized to apply a tensile force to the cover and to the fixation element.

According to some embodiments, a tension in the elastic tube ranges between approximately 1 gram to 400 grams.

According to some embodiments, a tension in the elastic tube ranges between approximately 1 gram to 200 grams.

According to some embodiments, a tension in the elastic tube ranges between approximately 3 grams to 50 grams.

According to some embodiments, the stomal insert further comprises a cap for sealing an opening to the elastic tube.

According to some embodiments, the cap includes a shell for accommodating a waste content collection bag.

According to some embodiments, the cap includes a tab for removing the cap from the opening.

According to some embodiments, the elastic tube includes a material of durometer ranging from 5 to 50 shore A for providing the axial elasticity.

According to some embodiments, the stomal insert further comprises a cap for sealing an opening to the elastic tube.

According to some embodiments, the cap includes a shell for accommodating a waste content collection bag.

According to some embodiments, the cap includes a tab for removing the cap from the opening.

According to some embodiments, the elastic tube includes a material of durometer ranging from 5 to 50 shore A for providing the axial elasticity.

According to some embodiments, the elastic tube comprises a material of durometer ranging from 20 - 30 shore A.

According to some embodiments, a portion of the elastic tube is adapted to bend for allowing relative motion between the cover and the fixation element.

According to some embodiments, the elastic tube is collapsible for allowing peristaltic propelling of waste content.

According to some embodiments, the fixation element includes a material of a durometer ranging from 5 to 50 Shore A.

According to some embodiments, the fixation element is a balloon.

According to some embodiments, the balloon includes a toroidal shape.

According to some embodiments, the toroidal shape includes a ring with a non-circular cross-section.

According to some embodiments, the balloon includes a material of a durometer ranging from 3 to 7 Shore A.

According to some embodiments, the balloon includes a seamless exterior surface.

According to some embodiments, the fixation element is deformable in an axial direction when pressed against the abdominal wall for maintaining a pressure on the abdominal wall.

According to some embodiments, the pressure ranges from 1 to 200 mmHg.

According to some embodiments, the pressure ranges from 20 to 150 grams.

According to some embodiments, the pressure ranges from 20 to 100 grams.

According to some embodiments, the cover includes an elastic material of a durometer ranging from 5 to 50 Shore A.

According to some embodiments, the cover includes an elastic material of a durometer ranging from 20 to 40 Shore A.

According to some embodiments, the cover is adapted to elastically press against the abdominal wall.

According to some embodiments, the cover is deformable by 5 mm in an axial direction.

According to some embodiments, the cover is substantially mushroom-shaped.

According to some embodiments, the cover includes a shape which is adjustable to geometrical variations in abdominal wall thickness.

The stomal insert may comprise an inflation port for administering an expansion fluid for inflating the inflatable balloon.

The stomal insert may comprise an inflation lumen for connecting the inflation port with the balloon.

The elastic tube may include an attachment mechanism for attaching a waste content collection bag.

The attachment mechanism may include a snap-and-fit arrangement for securing the waste content collection bag to the elastic tube.

According to an aspect of some embodiments of the present invention, there is provided a method for preventing leakage from a stoma on an abdominal wall comprising applying tension to a cover and a fixation element on opposing sides of the stoma with a collapsible pliable tube wherein at least one of said cover, said fixation element and said tube is adapted to elastically deform for regulating a pressure applied by said fixation element on said abdominal wall due to said tensile force; and characterized by the pressure applied by said fixation element due to said tensile force decreasing to less than 10 mmHg when a colonic pressure is equal to or greater than approximately 10 mmHg The method may include the step of administering through an inflation port an expansion fluid for inflating the fixation element.

There may be provided a stomal insert cap comprising a shell and a waste content collection bag fittedly accommodated in the shell wherein the waste content collection bag is deployable from the shell upon sensing of an intra-colonic pressure of approximately 100 mmHg or greater.

The waste collection bag may be deployed through a removable cover.

There may be provided a stomal insert cap comprising a deformable cover for providing a visual indication of an intra-colonic pressure of approximately 100 mmHg or greater.

A single-component stomal insert may further comprise an integrally formed waste content collection bag.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced. In general, similar numbers are used for similar elements in different figures.

In the drawings:
Figure 1 schematically illustrates a stomal insert, according to an embodiment of the present invention;
Figure 2A schematically illustrates the stomal insert inserted in a stoma with an intestinal portion fitted over the stomal insert and attached to an abdominal wall exterior; according to an embodiment of the present invention;
Figure 2B schematically illustrates the stomal insert inserted in the stoma in a deformed state where the geometry of abdominal wall has undergone a variation due to a change in body posture or body movement, according to an embodiment of the present invention;
Figure 2C schematically illustrates the stomal insert inserted in the stoma and pressing against the abdominal wall sealing the stoma, under the influence of intra-colonic pressure, according to some embodiments of the present invention;
Figures 3A and 3B schematically show a side view of the stomal insert and a cross-sectional view A-A of a crimped elastic tube section, respectively, according to an embodiment of the present invention;
Figure 4A schematically illustrates a spring system model of the stomal insert, according to some embodiments of the present invention;
Figure 4B is a graphical representation illustrating design goal values for a pressure exerted by a balloon in the stomal insert, optionally determined using the model of spring system, according to some embodiments of the present invention;
Figure 5A schematically illustrates the interplay in the deformation of the balloon and the elastic tube in a first scenario wherein the elastic component of the pressure exerted by the balloon is due to a tensile force predominating over the intra-colonic pressure component, according to some embodiments of the present invention;
Figure 5B schematically illustrates the interplay in the deformation of the balloon and the elastic tube in a second scenario wherein the intra-colonic pressure component of the pressure exerted by the balloon predominates over the elastic component, according to some embodiments of the present invention;
Figure 6A schematically illustrates the interplay in the deformation of the elastic cover and the elastic tube in a first scenario wherein the user is in the relaxed state, according to some embodiments of the present invention;
Figure 6B schematically illustrates the interplay in the deformation of the elastic cover and the elastic tube in a second scenario wherein user movement results in a variation in the abdominal geometry, according to some embodiments of the present invention;
Figure 7A schematically illustrates the interplay in the deformation of the elastic cover, the balloon, and the elastic tube, in a first scenario wherein the user is in the relaxed state, according to some embodiments of the present invention;
Figure 7B schematically illustrates the interplay in the deformation of the elastic cover, the balloon, and the elastic tube, in a second scenario wherein user movement results in variations in the abdominal geometry, according to some embodiments of the present invention;
Figure 8 schematically illustrates a stomal insert including an elastic cover, an elastic tube, an inflation port, and an inflation lumen, according to some embodiments of the present invention;
Figure 9 schematically illustrates a stomal insert including an elastic cover, an elastic tube, an inflation port, and an inflation lumen, according to some embodiments of the present invention;
Figures 10A and 10B schematically illustrate a stomal insert adapted to be inserted in stomas formed in abdominal walls varying in thickness from one user to another, according to some embodiments of the present invention;
Figures 11A - 11C schematically illustrate different embodiments of stomal inserts similar to the stomal insert of Figure 1 having different elastic stomal covers, respectively, according to some embodiments of the present invention;
Figure 12A schematically illustrates a stomal insert including an elastic stomal cover, an inflatable balloon, an elastic tube, an inflation port, and an inflation lumen, all integrally formed as a single component suitable for one-time use, according to some embodiments of the present invention;
Figures 12B and 12C show alternative embodiments for a port including an integral pump, in accordance with exemplary embodiments of the invention;
Figure 13 schematically illustrates a stomal insert with a removable cap for removing waste content, according to some embodiments of the present invention;
Figure 14 schematically illustrates a stomal insert with a built-in collection bag for collecting waste content, according to some embodiments of the present invention;
Figures 15A - 15C schematically illustrate a stomal insert including an attachment mechanism at a proximal end of an elastic tube, for attaching a collection bag, according to some embodiments of the present invention;
Figures 16A - 16C schematically illustrate a stomal insert including an attachment mechanism at a proximal end of an elastic tube, for attaching a cap including a collection bag accommodated inside a shell, according to some embodiments of the present invention;
Figures 17A - 17D schematically illustrate a stomal insert including an attachment mechanism at a proximal end of an elastic tube, for attaching a collection bag used in domestic applications, according to some embodiments of the present invention;
Figure 18 schematically illustrates a stomal insert including an elastic stomal cover adjustable for use with users of variable abdominal wall thickness, according to some embodiments of the present invention;
Figures 19 and 20A - 20C schematically illustrate a stomal insert including an elastic stomal cover adjustable for use with users of variable abdominal wall thickness, according to some embodiments of the present invention;
Figure 21 is a flow chart showing operation of the stomal insert, according to some embodiments of the present invention;

### DESCRIPTION OF SPECIFIC EMBODIMENTS OF THE INVENTION

The present invention, relates to an Ostomy port for use following Ostomy cases such as Colostomy, Ileostomy or Urostomy, or for fecal incontinence.

An aspect of some embodiments of the present invention relates to a flexible and elastic stomal insert. The stomal insert is advantageously configured to substantially prevent leakage of waste content and associated odors through a stoma, independently of variations in a user's abdominal geometry.

Optionally, the stomal insert substantially prevents leakage of waste content and associated odors independently of variations in an intra-colonic pressure in the user's intestinal portion, which may vary from 0 - 150 mmHg. Optionally, the stomal insert exerts a pressure on the intestinal portion equal to, or less than, the intra-colonic pressure when the intra-colonic pressure increases, substantially preventing compression-related injuries such as, for example, ischemia or necrosis. Geometric variations in the user's abdomen occur for any type of body movement, from minor movements associated with breathing up to major variations associated with activities such as, for example, practicing sports or bending over.

In an exemplary embodiment, the stomal insert is formed from three, elastic sections, an elastic stomal cover adapted to apply an axial force to an exterior of the user's abdomen for pressing the cover to the abdomen, an inflatable balloon adapted to exert an axial pressure to the visceral side of the abdomen pressing the balloon to the abdomen, and a pliable, axially elastic tensiled tube passing through the stoma and interconnecting the elastic cover with the balloon.

In some embodiments, the inflatable balloon is replaceable by any fixation element, elastic or non-elastic, or any combination thereof, suitable for axially pressing the intestinal portion against the abdominal wall while maintaining the sealing. Optionally, the elastic cover is replaceable by any cover, elastic or non-elastic, or any combination thereof, suitable for covering the stoma, and for pressing against the external abdominal wall.

In an exemplary embodiment, the three sections form part of an elastic system which continuously maintains the seal between the insert and the intestinal portion, and prevents the insert from applying excessive pressure on body parts (e.g. abdominal wall, intestinal portion) with each component elastically deforming responsive to variations in the abdominal geometry and/or the intra-colonic pressure. Alternatively, the elastic system includes only one elastic component, the elastic tube. Alternatively, the two elastic components are the balloon and the elastic tube.

According to some embodiments of the present invention, the cover and the balloon are pressed against the abdomen by a tensile (pulling) force exerted on each one of the components by the elastic tube when the intra-colonic pressure is substantially zero. Optionally, this attachment is independent of abdominal geometric variations. Optionally, this tensile force causes the balloon to exert a pressure on the abdominal wall in a range between 1 mmHg and 50 mmHg, inclusively, when the intra-colonic pressure is substantially zero. Optionally, the pressure exerted by the balloon is in a range between 3 mmHg and 40 mmHg, 7 mmHg and 25 mmHg, 12 mmHg and 18 mmHg. When the intra-colonic pressure is greater than zero, the increased intra-colonic pressure presses the balloon into the abdominal wall further securing the seal. The increase in the pressure exerted by the balloon on the abdominal wall is compensated by a decrease in the tensile force of the elastic tube as the balloon pushes the tube in a proximal direction away from the abdominal cavity. Optionally, the tensile force decreases to zero.

In some exemplary embodiments, the pressure exerted by the balloon against the abdominal wall is the same, or less than, the intra-colonic pressure. Optionally, a maximum pressure exerted by the balloon on the abdominal wall, due to the intra-colonic pressure, is in a range between 150 mmHg and 200 mm Hg. Optionally, the pressure exerted by the balloon on the abdomen when the intra-colonic pressure is greater than zero is substantially equal to, or less than, the intra-colonic pressure and is less than or equal to 200 mmHg, less than or equal to 150 mmHg, less than or equal to 100 mmHg, less than or equal to 80 mmHg, less than or equal to 50 mmHg, less than or equal to 40 mmHg, less than or equal to 25 mmHg. The above pressure ranges for the balloon pressing the intestinal portion against the abdominal wall are for a relatively reduced period of time during which the intra-colonic pressure is greater than zero, and substantially prevents compression-related injuries to the intestinal portion while providing sealing.

In an exemplary embodiment, the stomal insert includes a cap which is attached to the cover for sealing a proximal opening to the elastic tube. Optionally, sealing the opening to the elastic tube allows for an intra-colonic pressure buildup in the stomal insert. Optionally, the elastic tube extends outwards from the stoma due to the intra-colonic pressure buildup inside the elastic tube when the proximal opening is sealed by the cap, possibly further pulling the balloon against the abdominal wall. Optionally, the elastic tube extends outwards from the stoma due to pressure exerted by waste content inside the elastic tube. Optionally, the cover separates from and ceases to press on the abdomen due to the elastic tube extending outwards from the stoma.

According to some embodiments of the present invention, the elastic tube is bendable for allowing relative motion between the cover on the external side of the abdomen and the balloon on the visceral side of the abdomen during variations in the abdominal geometry. Optionally, the mechanical movement of the stomal insert substantially minimally interferes with tissue movement. Optionally, tissue movement in the various layers of the abdominal wall due to the mechanical movement of the stomal insert may allow greater user comfort. Optionally, bending of the elastic tube may contribute to preventing inadvertent stomal insert extraction during increased intra-colonic pressure as the pressure acts off-axis relative to the elastic cover. Additionally, the elastic tube crimps when an external force is radially applied for reducing pressure on a stomal portion of the abdomen. Optionally, the external force is that applied by the abdominal wall on a stoma having an inserted tube ranging in diameter from 10 mm - 30 mm. The crimping of the elastic tube increases the abdominal retention of the stomal insert as a greater tissue area in the stomal portion of the abdomen resists its withdrawal. Additionally, the elastic tube allows for peristaltic propelling of the waste contents.

According to some embodiments of the present invention, the inflatable balloon includes a toroidal shape. Alternatively, the balloon has a toroidal shape with a ring with a non-circular cross-section. Optionally, the cross-section of the ring is elliptical.

Alternatively, the balloon is elliptically shaped. Optionally, the balloon is configured for use with varying abdominal wall thicknesses (each user has a different abdominal wall thickness) such that a partially inflated balloon is suitable for use with relatively thick abdominal walls while a fully inflated balloon is suitable for use with relatively thin abdominal walls. Optionally, the balloon is deformable and shifts in an axial direction away from the abdomen for reducing the pressure on the abdomen as the intra-colonic pressure increases and the balloon is pressed against the abdominal wall. Additionally, the balloon is inflated with an expansion fluid, which may be a compressible fluid such as air. Optionally, the expansion fluid is any other type of biocompatible fluid suitable for inflating the balloon. Alternatively, the balloon includes a biocompatible expansion fluid which heats up with body temperature and causes the balloon to expand.

According to some embodiments of the present invention, the stomal insert includes an inflation port for administering the expansion fluid to the balloon. Optionally, a needleless syringe type device containing the expansion fluid is connected to the inflation port. Additionally, the stomal insert includes an inflation lumen interconnecting the inflation port with the balloon through which the expansion fluid flows for inflating the balloon.

According to some embodiments of the present invention, the shape of the balloon forms a funnel-shape at the distal end of the stomal insert. Optionally, the funnel shape assists in guiding fecal matter into the elastic tube as it is propelled towards the stoma, opening the tube at the crimped section. Optionally, an internal surface of the elastic tube and the balloon opening are preferably as smooth as possible for providing substantially undisturbed flow of the waste content. Surface smoothness may be enhanced by methods known in the art, for example surface treatment to the tooling in which the components are fabricated, inserting additives to the raw material, or coating the material's surface with smoothening coating such as, for example, parylene.

According to some embodiments of the present invention, the elastic stomal cover is adapted to elastically press against the exterior of the abdomen and cover the stoma. Optionally, the elasticity is such that substantially any point on a circumference of the elastic cover may be displaced in the axial direction by up to 10 mm, 8 mm, 6mm, 5mm, or less. Responsively, the pressure variation exerted by the balloon is no more than the pressure that is normally exerted by the balloon when the intra-colonic pressure is zero, thereby maintaining the sealing. Optionally, the elastic cover is mushroom-shaped. Optionally, the elastic cover is adjustable in size for accommodating to variable abdominal wall thicknesses.

An aspect of some embodiments of the present invention relates to an integrally formed, single-component stomal insert. Optionally, the single-component stomal insert is readily and inexpensively produced, and is suitable for one-time use (remove and throw away). For example, the single-component stomal insert is removed and replaced once per day, twice per day, three times per day, once every two days, once every three days, or more. Optionally, the single-component stomal insert includes one or more of other components such as built-in collection bags, collection bag attachment mechanisms, removable cap attachment mechanisms, inflation ports, inflation lumens, and the like.

According to some embodiments of the present invention, the sections of the stomal insert include materials of durometer ranging from 1- 50 Shore A. For example, the elastic tube may include material of durometer ranging from 1 - 50 Shore A, 5 - 50 Shore A, 10 - 40 Shore A, 20 - 30 Shore A. The inflatable balloon may include material of durometer ranging from 1 - 50 Shore A, 2-40 Shore A, 3 - 20 Shore A, 3-10 Shore A, 3 - 7 Shore A. The elastic cover may include material of durometer 1 - 50 Shore A, 5 - 50 Shore A, 10 - 40 Shore A, 20 - 40 Shore A. Optionally, the materials are non-abrasive and biocompatible, and may include, for example, silicone rubber, natural rubber, or other elastomeric and/or polymeric materials. Optionally, sections are of a thickness for substantially blocking odor transmissions. Optionally, the thickness is at least 0.50 mm. Optionally, the thickness is at least 0.60 mm, at least 0.70 mm, at least 0.80 mm, or more. Optionally, the sections may have a material thickness less than 0.50 mm, for example, less than 0.40 mm, less than 0.30 mm, less than 0.20 mm, or less. Optionally, the sections may include a dedicated coating adapted to reduce wall permeability for substantially blocking odor transmissions, for example parylene. Optionally, the single- component stomal insert includes sections with material thickness less than 0.50 mm. Optionally, the sections are seamless and have no parting lines, or include unexposed seams or parting lines, for preventing injury to bodily tissue due to abrasion with the seams and/or undesired concentrated mechanical stress along the seams/parting lines.

An aspect of some embodiments of the present invention relates to a collection bag which attaches to the stomal cover for the collection of waste content. Optionally, the collection bag includes a gas filter mechanism. Optionally, the bag is accommodated, for example by furling or folding inside the proximal portion of the elastic tube and is deployed from inside the elastic tube by the user and/or by the pressure of the waste content. Alternatively, the bag is included in the cap and is accommodated in the proximal portion of the elastic tube when the cap is attached to the cover. Optionally, the collection bag is an ostomy pouch known in the art. Optionally, the collection bag is of an elastic material. Alternatively, the collection bag is of a similar material to that of the ostomy pouch. Alternatively, the collection bag is a domestic bag, for example, a sandwich bag or a food storage bag, and is attached to the stomal insert by an attachment mechanism on the proximal portion of the elastic tube which includes a snap-fit arrangement, or other suitable arrangement known in the art.

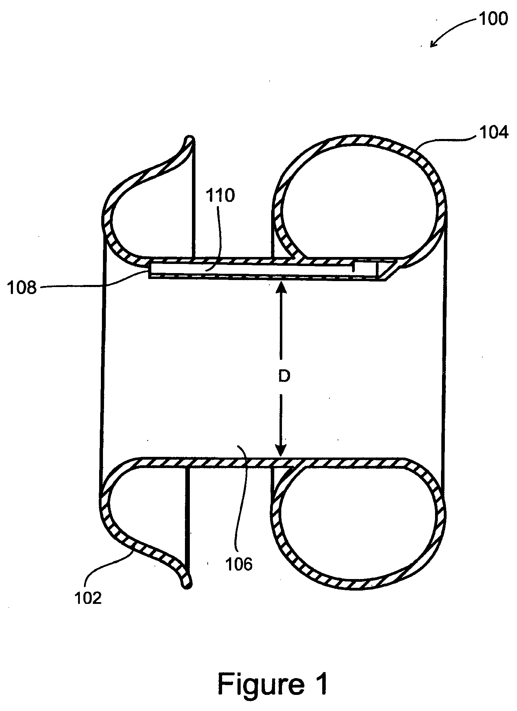

Referring now to the drawings, Figure 1 illustrates a stomal insert **100** according to an embodiment of the present invention. Stomal insert **100** includes a stomal cover **102** for covering the stoma from an external side of an abdominal wall (from outside the body), a fixation element **104** inserted in an intestinal portion inside an abdominal cavity for fixating the stomal insert relative to the intestinal portion and for preventing its removal through the stoma, and an axially elastic tube **106** of diameter D interconnecting the cover and the fixation element. Optionally, the diameter D is in the range between 10 - 30 mm, for example, 15 mm, 20mm, 25 mm. Stomal cover **102** is mushroom-shaped and may include an elastic material such as, for example, silicon, allowing the cover deformation flexibility. Fixation element **104** is a toroidal-shaped inflatable balloon. Stomal insert **100** includes an inflation port **108** through which an expansion fluid for inflating balloon **104** is administered, the expansion fluid flowing from the inflation port into the balloon through an inflation lumen **110.** Deflating of balloon **104** is also done by drawing out the expansion fluid from the balloon through the inflation lumen **110** and out the inflation port **108.** Inflation port **108** may include a Luer activated valve. Optionally, balloon **104** is self-inflatable and includes a biocompatible expansion fluid for inflating the balloon when inside the abdominal cavity. Optionally, balloon **104** is inflated by other methods known in the art not requiring use of inflation port **108** and/or inflation lumen **110.**

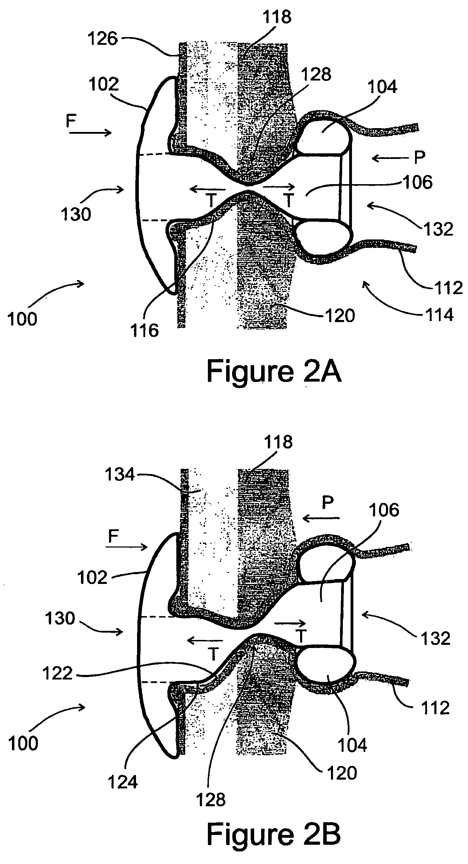

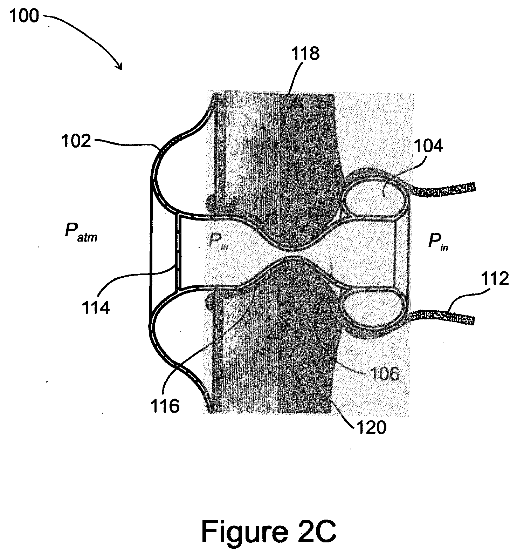

Referring also to Figures 2A, 2B, and 2C, stomal insert **100** is an ostomy port and serves to conduct waste contents such as fecal matter from an intestinal portion **112** in an abdominal cavity **114** of a user out a stoma **116** in an abdominal wall **118.** In some exemplary embodiments, stomal insert **100** is inserted through the stoma **116** into the intestinal portion **112,** with an interior surface **122** of the intestinal portion sealingly fitted over balloon **104** and an external surface **124** of elastic tube **106,** for substantially preventing leakage of waste contents and odor escape through stoma **116.** The seal between interior surface **122** and external surface **124** is maintained regardless of variations in geometry of abdomen **118** and/or of intra-colonic pressure in intestinal portion **112.** Optionally, the waste contents include urinary matter, intestinal portion **112** distally connected to a ureter in the user as a result of a urostomy.

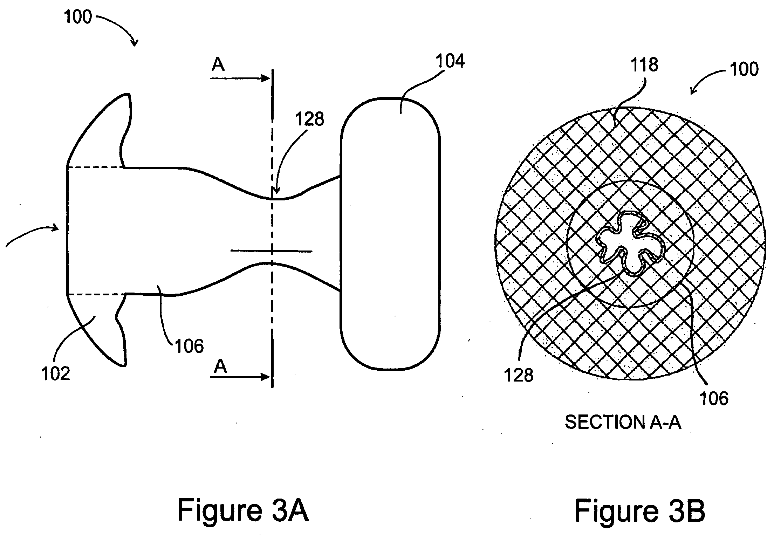

In Figure 2A, stomal insert **100** is shown inserted in stoma **116,** intestinal portion **112** fitted over the stomal insert and attached to an abdominal wall exterior **126.** Optionally, abdominal wall **118** is in a relaxed state and with an intra-colonic pressure of substantially 0 mmHg in intestinal Portion **112.** Optionally, in the relaxed state, a proximal opening **130** in stomal insert **100** is axially aligned with a distal opening **132.** Balloon **104** is inflated viscerally to a muscular layer **120** of the abdominal wall **118** and exerts a pressure **P** on intestinal portion **112** pressing the intestinal Portion against the muscular layer for providing the seal. Additionally, balloon **104** prevents inadvertent withdrawal of stomal insert **100** from stoma **116.** Optionally, cover **102** covers stoma **116** and presses against a side of exterior abdominal wall **126** with a force **F.** Optionally, cover **102** prevents the stomal insert from being drawn into stoma **116.** Elastic tube **106** applies a tensile force **T** on both cover **102** and balloon **104** pulling the cover against exterior abdominal wall **126** with the force **F** and the balloon against muscular layer **120** applying the pressure **P.** Optionally, an elastic tube section **128** inside stoma **116** is crimped by a radial pressure applied to the tube section by surrounding tissue in abdominal wall **118.** An example of the crimping of elastic tube **106** is shown in Figures 3A and 3B which show a side view of stomal insert **100** and a cross-sectional view A-A of crimped elastic tube section **128,** respectively, according to an embodiment of the present invention.

In Figure 2B, stomal insert **100** is shown inserted in stoma **116** in an deformed state, the geometry of abdominal wall **118** optionally having undergone a variation due to a change in body posture or body movement. An abdominal fat layer **134** has moved relative to abdominal muscular layer **120,** causing elastic cover **102** to move relative to balloon **104.** Optionally, elastic tube **106** bends to accommodate the relative movement of fat layer **134,** displacing proximal opening **130** relative to distal opening **132** so that they are no longer axially aligned. Optionally, elastic tube section **128** remains crimped under the pressure exerted by the surrounding tissue of abdominal wall **118.**

In Figure 2C, stomal insert **100** is shown inserted in stoma **116** pressing intestinal portion **112** against abdominal muscular layer **120** due to intra-colonic pressure, **P**ᵢₙ, according to some embodiments of the present invention. Stomal insert includes a cap **114** attached to cover 102 for sealing the proximal end of elastic tube **106.** Optionally cap **114** is removable or non-removable. Optionally, the pressure inside the elastic tube **107** is the intra-colonic pressure **P**ᵢₙ. Optionally, as **P**ᵢₙ increases and there is peristaltic contractions of intestinal portion **112, P**ᵢₙ increases above the atmospheric pressure **P**ₐₜₘ outside abdominal wall **118.** Optionally, the larger **P**ᵢₙ pushes stomal insert **100** in a proximal direction out of stoma **116,** and is counteracted upon by abdominal muscular layer **120** pushing on the balloon. Optionally, intestinal portion **112** also counteracts stomal insert **100** being pushed in the proximal direction. A larger difference between **P**ᵢₙ and **P**ₐₜₘ results in a better sealing as balloon **104** is pressed further into muscular layer **120.**

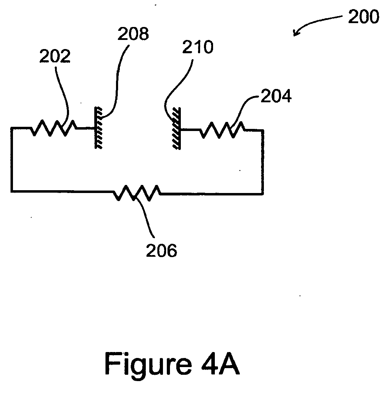

The stomal insert **100** may be modeled by a spring system **200** having three springs **202, 204,** and **206** as schematically shown in Figure 4A. Optionally, cover **102** is elastic and may be represented by spring **202,** inflatable balloon **104** by spring **204,** elastic tube **106** by spring **206,** exterior abdominal wall **126** by fixed support **208,** and muscular layer by fixed support **210.** Optionally, interplay exists between springs **202 - 206,** each one deforming independently to allow spring system **200** to maintain a predetermined range of pressures applied on fixed supports **208** and **210.** Optionally, using the model of spring system **200,** an amount of deformation may be determined for elastic cover **102,** inflatable balloon **104,** and elastic tube **106,** for allowing **P** to be maintained in the predetermined range between 1 mmHg and 50 mmHg in the relaxed state with intra-colonic pressure substantially zero, and less than 200 mmHg in an deformed state where there are variations in abdominal geometry and/or when the intra-colonic pressure is greater than zero.

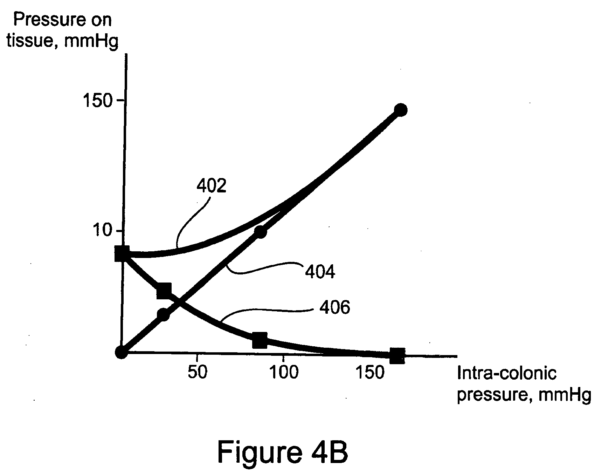

Figure 4B is a graphical representation illustrating design goal values for **P** using the model of spring System **200.** Solid curve **402** represents the force **F2** exerted on muscular layer **120** by balloon **104,** round curve **404** represents a pressure component of **P** due to intra-colonic pressure, and square curve **406** the elastic component of **P** due to the tensile force **T** in elastic tube **106.** When the intra-colonic pressure is zero, sealing is substantially maintained due to the tensile force exerted by elastic tube **106** on balloon **104** and elastic cover **102.** In case of low non-zero intra-colonic pressure (on the order of few mmHg), two mechanisms are contributing to balloon **104** exerting pressure **P** against muscular layer **120,** the tensile force from elastic tube **106** (elastic component) and the intra-colonic pressure in intestinal portion **112** (intra-colonic pressure component). As the intra-colonic pressure further increases, stomal insert **100** is pushed outwards. As a result, the intra-colonic pressure component of **P** increases, whereas **F** exerted on the exterior abdominal wall **126** by elastic cover **102** is relieved and the elastic component of **P** decreases.

In some embodiments, there is a level of intra-colonic pressure at which elastic cover **102** bulges out from exterior abdominal wall **126.** The elastic component of **P** is zero, and sealing is maintained solely due to the intra-colonic pressure component of **P.** Any elevation in the intra-colonic pressure increases the pressure exerted by balloon **104** against muscular layer **120,** and further secures the seal. Upon relief of the intra-colonic pressure the elastic nature of elastic cover **102,** balloon **104,** and elastic tube **106,** return stomal insert **100** to its relaxed state.

In some embodiments, the elasticity of elastic cover **102,** balloon **104,** and elastic tube **106,** maintains the seal between stomal insert **100** and intestinal portion **112** substantially throughout a whole range of intra-colonic pressures. Low intra-colonic pressure sealing is contributed to mainly by elasticity; the elastic component vanishing at higher pressure levels. Optionally, balloon **104** pressure on muscular layer **120** is maintained at a relatively low value, and a risk of compression-related tissue damage such as ischemia, necrosis, and the like is hence substantially reduced.

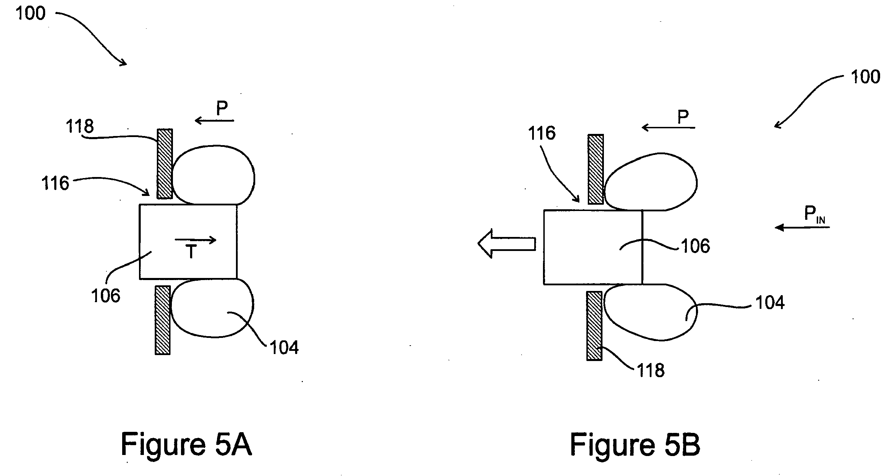

Reference is made to Figures 5A and 5B which schematically illustrate the interplay in the deformation of balloon **104** and elastic tube **106** in a first scenario wherein the elastic component of **P** due to the tensile force **T** predominates over the intra-colonic pressure component (intra-colonic pressure is zero or relatively small), and in a second scenario wherein the intra-colonic pressure component of **P** due to the intra-colonic pressure **P**ᵢₙ predominates over the elastic component, respectively, according to some embodiments of the present invention. In Figure 5A, stomal insert **100** is inserted through stoma **116** in abdominal wall **118.** Optionally, elastic tube **106** applies the tensile force **T** to balloon **104** pulling the balloon and pressing it to abdominal wall **118.** In Figure 5B, the intra-colonic pressure pushes balloon **104** against abdominal wall **118.** Optionally, balloon **104** partially deforms in an axial direction away from abdominal wall **118** for reducing the pressure **P** exerted on the abdominal wall. Optionally, elastic tube **106** is partially pushed out of stoma **116** in a proximal direction by the pressing of balloon 104 on abdominal wall **118** so that the tensile force **T** applied to the balloon is reduced to substantially zero. Optionally, only the intra-colonic pressure component of **P** acts on abdominal wall **118.** Optionally, the elastic cover (not shown) bulges out from abdominal wall **118.**

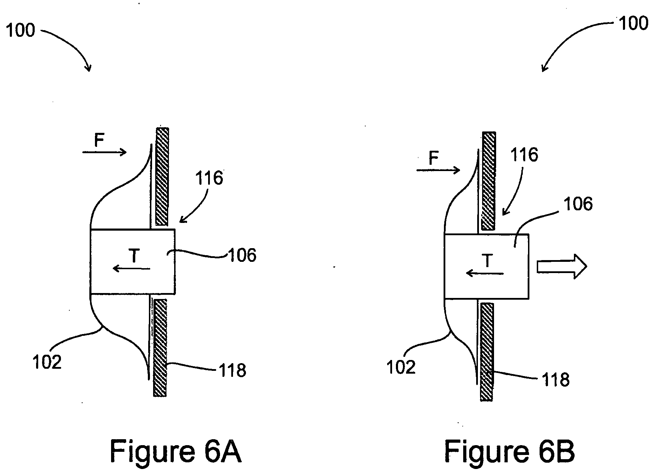

Reference is made to Figures 6A and 6B which schematically illustrate the interplay in the deformation of elastic cover **102** and elastic tube **106** in a first scenario wherein the user is in the relaxed state, and a second scenario wherein user movement results in a variation in the abdominal geometry, respectively, according to some embodiments of the present invention. In Figure 6A, elastic tube **106** applies the tensile force T to elastic cover **102** for pressing the elastic cover against abdominal wall **118.** Optionally, elastic cover **102** presses against abdominal wall **118** with force **F** = **T.** In Figure 6B, the variation in the abdominal geometry results in elastic tube **106** being pulled in stoma **116** in a distal direction (into the abdominal cavity). Optionally, the tensile force **T** increases further pulling on elastic cover **102** which presses on abdominal wall **118** and partially deforms (for example, partially flattens) for reducing the pressure exerted on the abdominal wall.

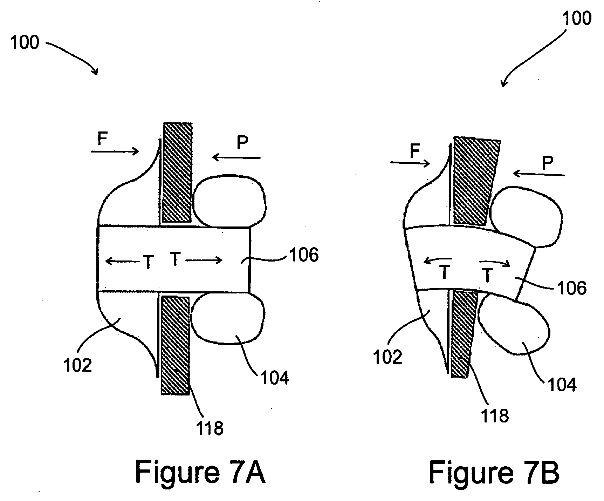

Reference is made to Figures 7A and 7B which schematically illustrate the interplay in the deformation of elastic cover **102,** balloon **104,** and elastic tube **106,** in a first scenario wherein the user is in the relaxed state, and a second scenario wherein user movement results in variations in the abdominal geometry, according to some embodiments of the present invention. In Figure 7A, elastic tube **106** applies the tensile force **T** pressing elastic cover **102** and balloon **104** against abdominal wall **118.** Optionally, elastic cover **102** and balloon **104** press against abdominal wall **118** with force **F = T** and **P** = **T/A** where A is a surface area of balloon **104** in contact with the abdominal wall, respectively. In Figure 6B, the variations in the abdominal geometry optionally result in bending of elastic tube **106** so that the axial tensile force **T** acts on elastic cover **102** by deforming some portions of the cover more than others. Additionally, tensile force **T** acts on balloon **104** by optionally deforming along the axial direction some portions of the balloon more than others for maintaining the balloon pressing against abdominal wall **118** at the predetermined range of pressures.

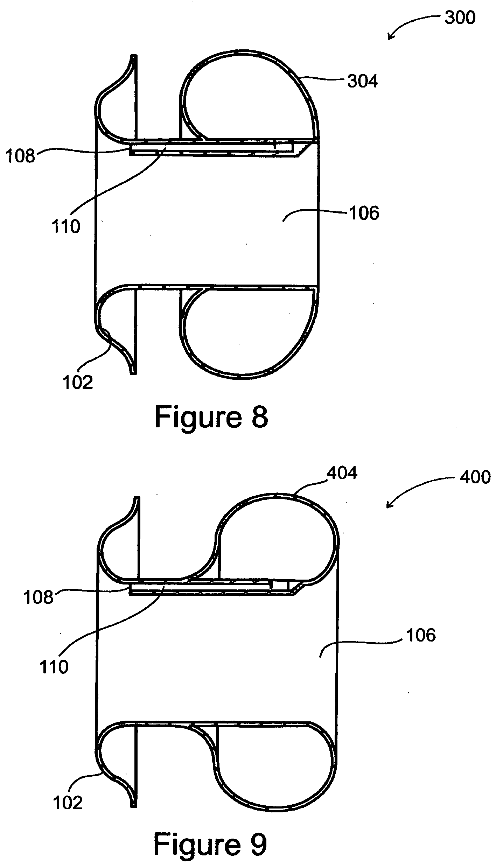

Figure 8 schematically illustrates a stomal insert **300** including elastic cover **102,** elastic tube **106,** inflation port **108** and inflation lumen **110,** according to some embodiments of the present invention. Optionally, stomal insert **300** additionally includes balloon **304** functionally similar to balloon **104** in Figure 1 but having a toroidal shape with a ring including a non-circular cross-section. Optionally, balloon **304** is assembled in stomal insert **300** such that the stomal insert or its components have no exposed seams or parting lines which may contribute to tissue damage.

Figure 9 schematically illustrates a stomal insert **400** including elastic cover **102,** elastic tube **106,** inflation port **108** and inflation lumen **110,** according to some embodiments of the present invention. Optionally, stomal insert **400** additionally includes balloon **404** functionally similar to balloon **104** in Figure 1 but having a toroidal shape with a ring including a non-circular cross-section. Optionally, balloon **404** is assembled in stomal insert **400** such that the stomal insert or its components have no exposed seams or parting lines which may contribute to tissue damage.

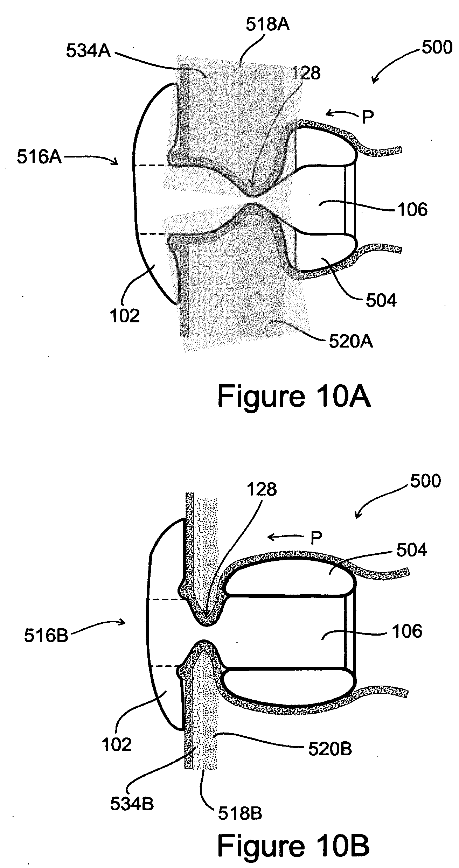

Figures 10A and 10B illustrate a stomal insert **500** adapted to be inserted in stomas formed in abdominal walls varying in thickness from one user to another, according to some embodiments of the present invention. Optionally, stomal insert **500** includes elastic cover **102,** elastic tube **106,** and an inflatable balloon **504** functionally similar to balloon **104** and which may be partially inflated or fully inflated to adjust for varying degrees of abdominal wall thickness.

In Figure 10A, stomal insert **500** is shown inserted through a stoma **516A** formed in an abdominal wall **518A** having a relatively thick fatty layer **534A** and a relatively thick muscular layer **520A**. Optionally, balloon **504** is partially inflated with an expansion fluid (not shown) and presses against the muscular layer **520A,** elastic tube section **128** crimped substantially along a mid portion of elastic tube **106.** Optionally, balloon **504** may press against muscular layer **520A** using the intra-colonic pressure component of **P,** the elastic component, or a combination of both.

In Figure 10B, stomal insert **500** is shown inserted through a stoma **516B** formed in an abdominal wall **518B** having a relatively thin fatty layer **534A** and a relatively thin muscular layer **520A.** Optionally, balloon **504** is fully inflated with the expansion fluid and presses against the muscular layer **520B,** elastic tube section **128** crimped substantially along a substantially forward portion of elastic tube **106.** Optionally, balloon **504** may press against muscular layer **520B** using the pressure component of the force **F2,** the elastic component, or a combination of both.

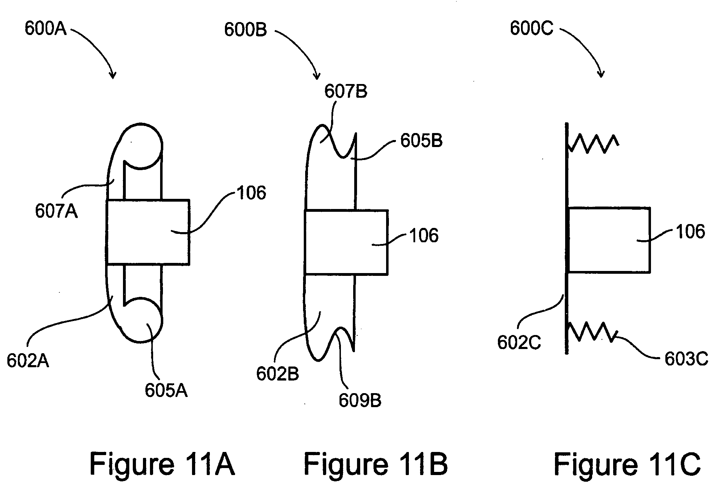

Figures 11A - 11C schematically illustrate different embodiments of stomal inserts **600A - 600C** similar to stomal insert **100** but having different elastic stomal covers **602A - 602C,** respectively, according to some embodiments of the present invention. Optionally, elastic covers **602A - 602C** are functionally similar to elastic cover **102** but are differently shaped. Optionally, in Figure 11A, elastic cover **602A** is circularly shaped having a circumferential rounded rim **605** for pressing against the abdominal wall and a deformable surface **607A** connected to elastic tube **106.** Optionally, in Figure 11B, elastic cover **602B** includes a circumferential edge **605B** for pressing against the abdominal wall and a deformable surface **607B** connecting to the elastic tube **106** and through a curved section **609B** to the circumferential edge for providing a degree of resiliency in the elastic cover. Optionally, in Figure 11C, elastic cover **602C** is a flexible plate connecting to elastic tube **106,** and includes springs **603C** for pressing against the abdominal wall. Alternatively, plate **602C** may be rigid or semi-rigid.

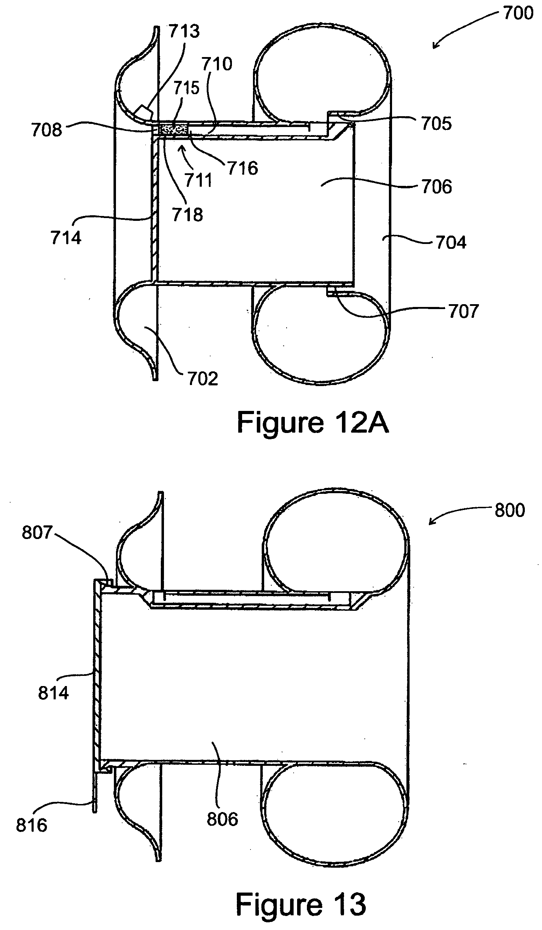

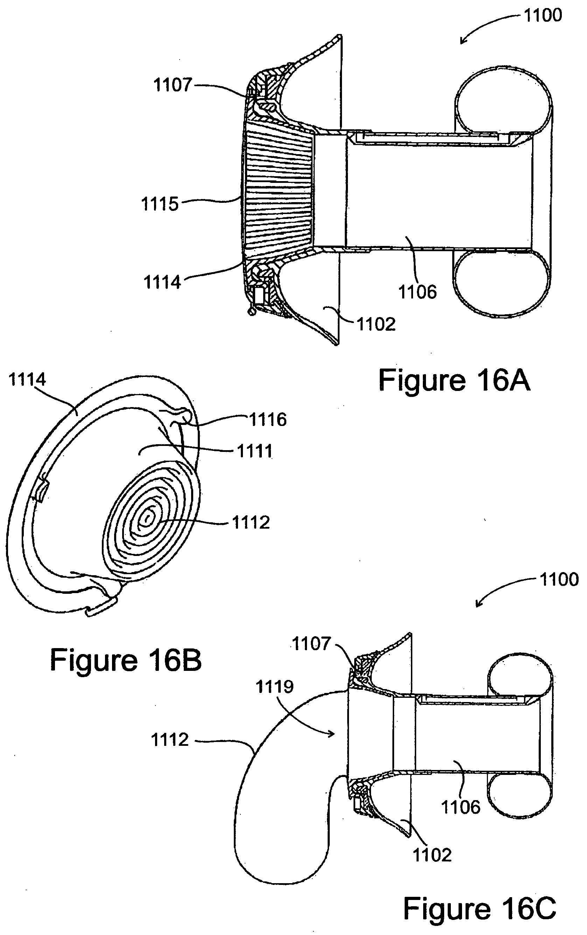

Figure 12A schematically illustrates a stomal insert **700** including an elastic stomal cover **702,** an inflatable balloon **704,** and an elastic tube **706** all integrally formed as a single component suitable for one-time use, according to some embodiments of the present invention. Optionally, stomal insert **700** includes an inflation port **708** and an inflation lumen **710.** Stomal insert **700,** including elastic cover **702,** balloon **704,** elastic tube **706,** inflation port **708,** and inflation lumen **710,** may be similar to that shown in Figure 1 at **100** including **102, 104, 106, 108,** and **110,** optionally, with the difference that in some embodiments balloon **704** is formed with a loose end **705** attachable to a distal exterior surface **707** of the elastic tube during assembly of the stomal insert. Optionally, a proximal end of elastic tube **706** is fitted with a non-removable cap **714** (or seal) so that stomal insert **700** is removed and disposed of when filled with waste content. In some embodiments, seal **714** is embodied as an expanding waste bag, for example, as described with respect to Figure 16A and 16B.

In an exemplary embodiment of the invention, an optional self contained pump **711** is provided. Such a pump **711** may also be provided with other port designs, for example, those that include a balloon or other inflatable elements.

In one example, pump **711** is an air pump. Optionally, pump **711** is located in line with inflation channel **710,** optionally within it. In an alternative embodiment, pump **711** is mounted on or integral with stomal cover **702.**

In an exemplary embodiment of the invention, the pump is an air pump which uses a foam or other expanding element **715** to expand and includes at least one one-way valve **716.** In operation, when the pump is compressed, air in the foam is pushed through valve **716** (e.g., a one way flap valve) into lumen **710.** Optionally, a second one way valve **718,** for example, a flap valve, is provided to prevent air escape during such compression. When the pump is released, the foam or other expanding element elastically expands, drawing air in from the outside. Balloon deflation is optionally provided by pressing on the valves so they are not patent. In an exemplary embodiment of the invention, the flaps are integrally formed with the body of the port. Alternatively the pump is attached, optionally using adhesive.

Such valves may be provided also without the pump mechanism, for example, in other embodiments of the invention.

In an alternative embodiment, a liquid pump is provided. Optionally, a compressible reservoir **713** is provided filled with liquid or other fluid and which is fluidically connected to lumen **710,** via a one way valve (e.g., **71**6). When reservoir **713** is compressed, fluid passes through the valve into lumen **710.** Optionally, manipulating the valve allows the retention balloon to deflate.

Optionally, the reservoir is large enough to fill the entire balloon. In an alternative embodiment, the reservoir is only large enough for manipulating the balloon pressure, for example, having a volume of, for example, between 10% and 50% thereof. Optionally, lumen **710** can be used for filling the balloon and/or reservoir as described herein.

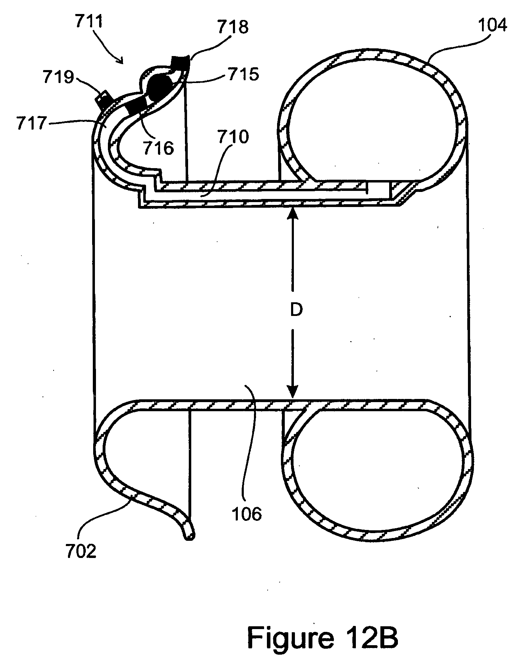

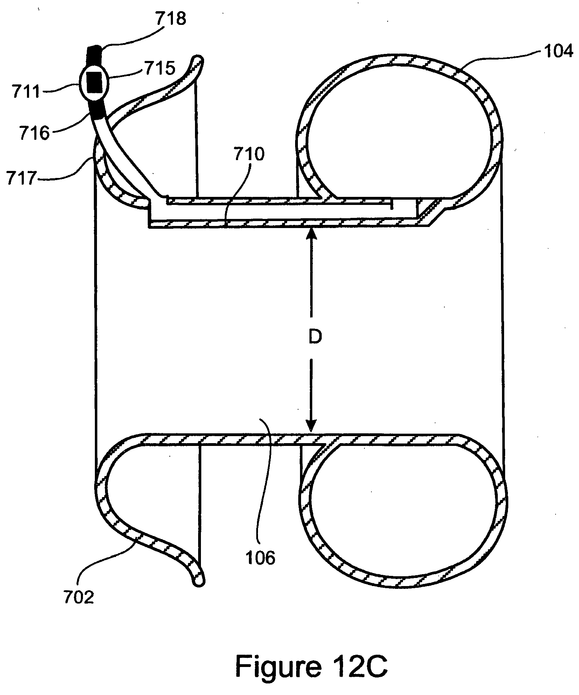

Figures 12B and 12C show alternative embodiments for a port including an integral pump **711,** in accordance with exemplary embodiments of the invention.

In figure 12B, an extension **717** to lumen **710** is provided along and/or integrated with stomal cover **702,** with pump **711** (e.g., using a foam element in a pump cavity and two one way valves), integral therewith in the cover. In use, the pump may be pinched between two fingers.

Figure 12B also shows an optional deflation valve **719,** which could also be located at other places along lumens **710** and/or **717** and/or attached using a separate or partially separate deflation lumen.

In figure 12C, extension lumen **717** may be a separate tube and pump **711** is provided at an end of the tube, with pump **711** optionally hanging separately from stomal cover **702.** Optionally, the pump can be attached to cover **702,** for example, using Velcro (e.g., a hook and loop connector) or a tack substance and/or placed between cover **702** and the body. Figure 13 schematically illustrates a stomal insert **800** with a removable cap **814** for removing waste content, according to some embodiments of the present invention.

Stomal insert **800** includes an elastic tube **806** with a cap attachment mechanism **807** for securing cap **814** to the stomal insert. Stomal insert **800** is similar to stomal insert **100** shown in Figure 1 except for removable cap **814** and cap attachment mechanism **807,** which may be as known in the art. Removable cap **814,** in some embodiments, has a tab **816** for easy removal of the cap. Optionally, attachment mechanism **807** is adapted to be fitted with a collection (not shown) into which waste content flows out of stomal insert 800 following removal of cap 814. Optionally, the collection bag is attached to attachment mechanism 807 following removal of cap 814. Optionally, stomal insert **800** is formed as a single component similar to stomal insert **700** shown in Figure 12, excluding removable cap **814** which is separately produced from the stomal insert.

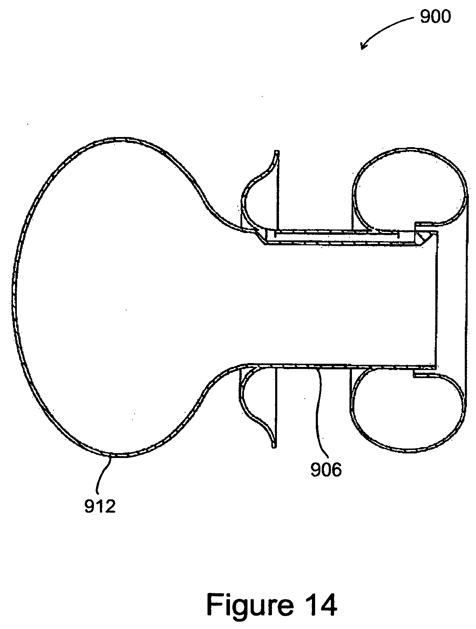

Figure 14 schematically illustrates a stomal insert **900** with a built-in collection bag **912** for collecting waste content. Stomal insert **900** includes an elastic tube **906** to which collection bag **912** is attached at a proximal end. Collection bag **912** is accommodated inside elastic tube **906.** Optionally, collection bag **912** may have any shape suitable for collecting waste content while stomal insert **900** is in use by a user. Collection bag **912** is attached to an attachment mechanism (not shown) on elastic tube **906.** Stomal insert **900** including elastic tube **906** is similar to stomal insert **100** including elastic tube 101 shown in Figure 1 except for the collection bag arrangement. Optionally, stomal insert **900** is formed as a single component similar to stomal insert **700** shown in Figure 12 with the difference of collection bag **912.**

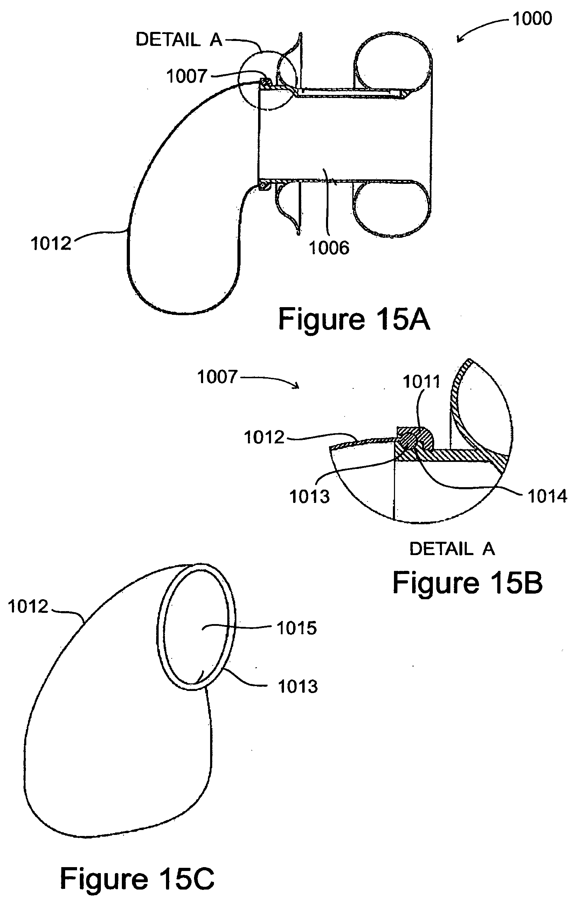

Figures 15A - 15C schematically illustrate a stomal insert **1000** including an attachment mechanism **1007** at a proximal end of elastic tube **1006,** for attaching a collection bag **1012.** Collection bag **1012** includes an opening **1015** with an annular elastic rim **1013** adapted to be inserted in a circumferential recess **1014** in attachment mechanism **1007.** Optionally, attachment mechanism **1007** includes a locking mechanism **1011** for locking annular elastic rim **1013** inside circumferential recess **1014,** and is adapted to support a weight of collection bag **1012** including waste content. Optionally, annular elastic rim **1013** includes a latex material or other type of elastomeric material. Alternatively, annular elastic rim is non-elastic and is preformed of a circumference suitable for fitting in circumferential recess **1014** and locked in place by locking mechanism **1011.** Optionally, collection bag **1012** includes an elastic material. Stomal insert **1000** including elastic tube **1006** is similar to stomal insert **100** including elastic tube **101** shown in Figure 1 except for attachment mechanism **1007.**

Figures 16A - 16C schematically illustrate a stomal insert **1100** including an attachment mechanism **1107** at a proximal end of elastic tube **1106,** for attaching a cap **1114** including a collection bag **1112** accommodated inside a shell **1111.** Cap **1114** is adapted to be attached to the proximal end of elastic tube **1106** such that collection bag **1112** is inside the elastic tube. Optionally, cap **1114** attaches to an attachment mechanism **1107** at a proximal end of elastic tube **1106,** and optionally provides an air-tight seal of the elastic tube. Optionally, the air-tight seal allows for an increase in the intra-colonic pressure in elastic tube **1106.** Optionally, attachment mechanism **1107** includes a "bayonet" locking arrangement for sealingly accommodating mating tabs **1116** on cap **1114.** Alternatively, attachment mechanism **1107** and cap **1114** include other types of lock mating arrangements known in the art. Stomal insert **1100** including elastic tube **1106** is similar to stomal insert **100** including elastic tube **101** shown in Figure 1 except for attachment mechanism **1107.**

Cap **1114** includes a proximal opening **1119** onto which is fitted a removable cover **1115,** the cover adapted to be removed by a user for allowing collection bag **1112** to be deployed through the opening for collecting waste content. Optionally, collection bag **1112** is deployed by the user and the waste content flows into the collection bag pushed by the intra-colonic pressure. Alternatively, collection bag **1112** is pushed out by pressure exerted by the waste content and/or the intra-colonic pressure. Optionally, removable cover **1115** is removed from cap **1114** by a pushing force exerted by collection bag **1112** due to pressure from the waste content and/or the intra-colonic pressure. Optionally, cap 1114 and/or removable cover 1115 is of a stretchable material which allows it to deform and protrude outwards due to pressure exerted by waste content and/or flatus inside elastic tube 1107. Optionally, the deformation of cap 1114 and/or cover 1115 serves to indicate to the user of a need to release gas or remove waste content.

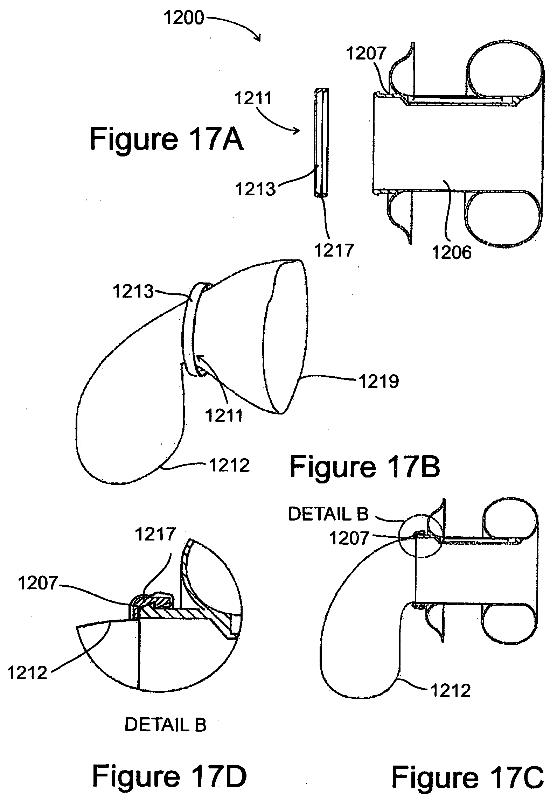

Figures 17A - 17D schematically illustrate a stomal insert **1200** including an attachment mechanism **1207** at a proximal end of elastic tube **1206,** for attaching a collection bag **1212** used in domestic applications (for example, a sandwich bag, small garbage bags, and the like). Optionally, attachment mechanism **1207** includes a snap-fitting arrangement for attaching a removable annular fastener **1213** having an opening **1211** through which a portion of collection bag is inserted. Optionally, annular fastener **1213** includes a latching element **1217** which snap-fits onto attachment mechanism **1207** and secures a rim portion **1219** of collection bag **1212** between the latching element and the attachment mechanism. Optionally, other fastening arrangements known in the art may be used for removable annular fastener 1213 and attachment mechanism **1207** for securing collection bag **1212** to stomal insert **1200.** These may include twist-locking and securing rim portion **1219** by pressing on the rim portion in an axial direction parallel to that of elastic tube **1206,** or using a spring mechanism in attachment mechanism **1207** so that the spring secures the rim portion while pressing against annular fastener **1213.** Stomal insert **1200** including elastic tube **1206** is similar to stomal insert **100** including elastic tube **101** shown in Figure 1 except for attachment mechanism **1207** and removable annular fastener **1213.**

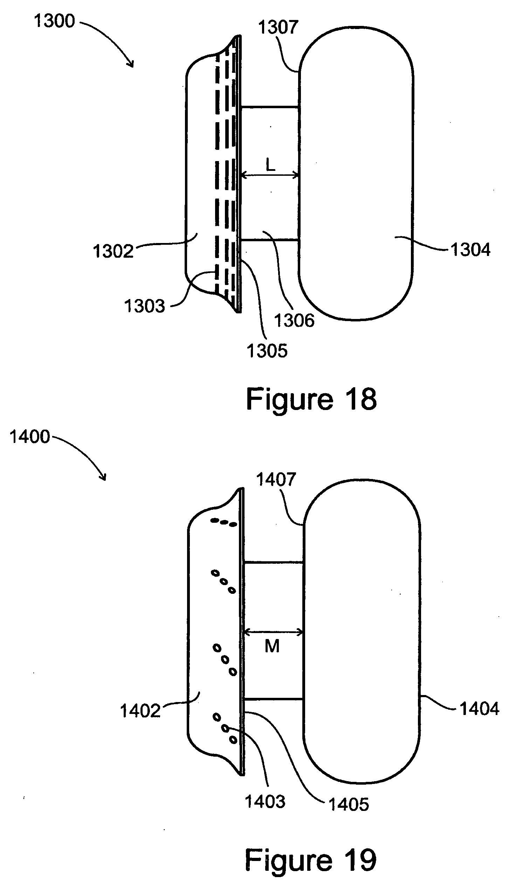

Figure 18 schematically illustrates a stomal insert **1300** including an elastic stomal cover **1302** adjustable for use with users of variable abdominal wall thickness, according to some embodiments of the present invention. Stomal cover **1302** includes cutting marks **1303** circumferentially arranged on the stomal cover and adapted to be cut in a circumferential direction prior to introducing stomal insert **1300** into a stoma, for increasing a distance L between a distal edge **1305** of the stomal cover of and a proximal end **1307** of a balloon **1304. L** is in a range between 1 cm - 15 cm, for example, 3 cm, 6 cm, 9 cm, 12 cm. Optionally, a length of elastic tube **1306** remains the same. Optionally, increasing distance L allows stomal insert **1300** to be accommodated in a thicker abdominal wall. Stomal insert **1300** including elastic cover **1302,** balloon **1304,** and elastic tube **1306,** may be similar to that shown in Figure 1 at **100,** including **102**, **104**, and **106.**

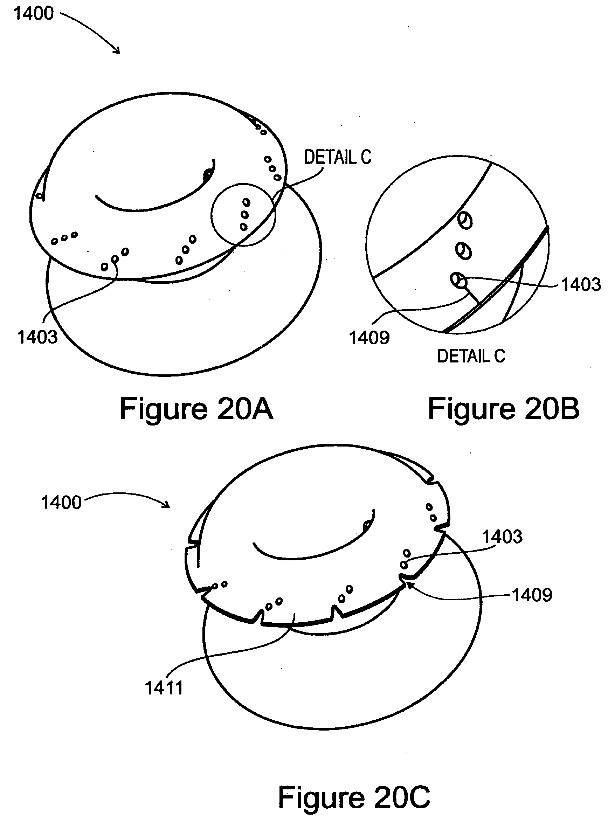

Figures 19 and 20A - 20C schematically illustrate a stomal insert **1400** including an elastic stomal cover **1402** adjustable for use with users of variable abdominal wall thickness. Optionally, elastic cover **1402** includes cutting marks **1403** circumferentially arranged along the stomal cover and adapted to be cut by radial cuts **1409** extending from a distal end **1405** of the stomal cover prior to introducing stomal insert **1400** in a stoma. Optionally, radial cuts **1409** separate elastic cover **1402** into sections **1411** capable of flexing in a proximal direction for increasing a distance M between distal edge **1405** and a proximal end **1407** of a balloon **1404.** Optionally, a length of elastic stomal tube **1406** remains the same. Optionally, increasing distance M allows stomal insert **1400** to be accommodated in a thicker abdominal wall. Stomal insert **1400** including elastic cover **1402,** balloon **1404,** and elastic tube **1406,** may be similar to that shown in Figure 1 at **100,** including **102, 104,** and **106.**

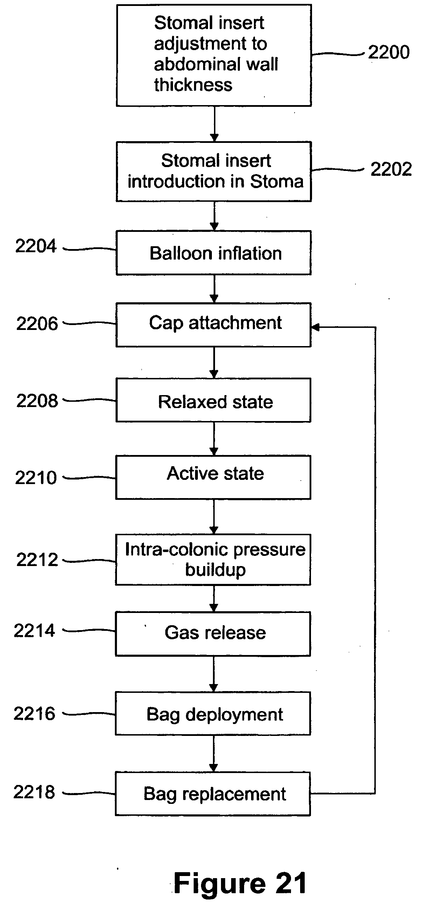

Figure 21 is a flow chart showing exemplary operation of stomal insert **100.**

At **2200,** stomal insert **100** is adjusted to a size required by a user. Optionally, a length of elastic tube **106** is cut to a user's length at a manufacturing facility of stomal insert **100** and the stomal insert is manufactured by attaching cover **102** and balloon **104** to the cut elastic tube. Alternatively, cover **102** and balloon **104** are integrally formed with elastic tube **106** such that the length of elastic tube **106** is preadjusted to that required by the user. Additionally or alternatively, the user fits stomal insert **100** to the required size by trimming stomal cover **102**

At **2202,** the user inserts stomal insert **100** into stoma **116** in a distal direction from the external abdominal wall 126 into the abdominal cavity until cover **102** is pressed against the external abdominal wall. Optionally, a physician or other appropriate medical personnel performs the insertion.

At **2204,** the user inflates the balloon by introducing an expansion fluid into inflation port **108.** Optionally, inflation port **108** includes a Luer connector to which the user attached a needleless syringe with the expansion fluid. Optionally, balloon **104** is self-expanding and includes an expansion fluid. Optionally, the expansion fluid is heat activated by the internal body heat and expands accordingly. Optionally, the expansion fluid expands after a predetermined period of time. Optionally, the balloon expands to a predetermined size, which may be fully expanded, or partially expanded.

At **2206,** the user optionally attaches a cap **114** to cover **102.** Optionally, cap **114** includes a collection bag which is accommodated in a proximal section of elastic tube **106.**

At **2208,** the user is in the relaxed state, and proximal opening **130** and distal opening **132** in stomal insert **100** are substantially aligned. Optionally, elastic tube **106** includes a crimped portion **128** where surrounding tissue in the abdominal wall **118** exerts a radial force on the elastic tube. Optionally, elastic tube 106 applies the tensile force **T** to balloon **104** so that the balloon presses on muscular layer **120** with a pressure **P** ranging from 1 mmHg to 50 mmHg. Optionally, only the elastic component of **P** exerts the pressure of balloon 104 (the intra-colonic pressure component is substantially zero).

At **2210,** the user is in the deformed state stomal insert **100** and abdominal fat layer **134** has moved relative to abdominal muscular layer **120,** causing elastic cover **102** to move relative to balloon **104.** Optionally, elastic tube **106** bends to accommodate the relative movement of fat layer **134,** displacing proximal opening **130** relative to distal opening **132** so that they are no longer axially aligned. Optionally, elastic tube section **128** remains crimped under the pressure exerted by the surrounding tissue of abdominal wall **118.** Optionally, elastic tube **106** applies the tensile force **T** to balloon **104** so that the balloon presses on muscular layer **120** with a pressure **P** ranging from 1 mmHg to 50 mmHg. Optionally, only the elastic component of **P** exerts the pressure of balloon **104** (the intra-colonic pressure component is substantially zero).

At **2212,** the user experience intra-colonic pressure buildup, the pressure in intestinal portion **112** and elastic tube **106** optionally, building up to 150 mmHg. Optionally, balloon **104** presses intestinal section **112** against muscular layer 120 with a pressure P less than or equal to 150 mmHg maintaining the sealing. Optionally, the elastic component and the intra-colonic pressure components of **P** combine to exert the pressure of balloon **104.** Optionally, the elastic component decreases to substantially zero and only the intra-colonic component influences the pressure exerted by balloon **104.**

At **2214,** the user optionally releases an active gas filtering mechanism for releasing flatus in elastic tube **106.** Optionally, cap **114** protrudes outwardly as an indication of the gas in elastic tube **106.** Optionally, the active gas mechanism is automatically activated by the increased intra-colonic pressure. Optionally, a passive gas filtering mechanism filters the flatus. At **2216,** the user opens cap **114** and pulls out a collection bag from inside elastic tube **106.** Optionally, the collection bag is pushed out of elastic tube **106** by the waste content in the elastic tube. Optionally, the collection bag is included in cap **114.** Optionally, the collection bag is pulled out through an opening in cap **114** following removal of a cap cover so that the cap does not require prior removal. Optionally, the bag is a domestic collection bag.

At **2218,** the user removes the collection bag with the waste content and replaces the bag accommodating it inside elastic tube 106. Optionally, the cap and the collection bag are both removed. Optionally, the user performs irrigation before replacing the bag. Return to **2206.** The terms "comprises", "comprising", "includes", "including", "having" and their conjugates mean "including but not limited to". This term encompasses the terms "consisting of" and "consisting essentially of".

The phrase "consisting essentially of means that the composition or method may include additional ingredients and/or steps, but only if the additional ingredients and/or steps do not materially alter the basic and novel characteristics of the claimed composition or method.

As used herein, the singular form "a", "an" and "the" include plural references unless the context clearly dictates otherwise. For example, the term "a compound" or "at least one compound" may include a plurality of compounds, including mixtures 25 thereof.

The word "optionally" is used herein to mean "is provided in some embodiments and not provided in other embodiments". Any particular embodiment of the invention may include a plurality of "optional" features unless such features conflict.

## Claims

1. An ostomy port (100) for conducting waste content from an intestinal portion in an abdominal cavity through a stoma in an abdominal wall comprising;
a cover (102) for covering the stoma;
a fixation element (104) for anchoring the port to a visceral side of the abdominal wall;
a pliable, axially elastic tube (106) interconnecting said cover (102) and said fixation element (104) and sized to apply a tensile force (406) to said cover (102) and to said fixation element (104);
**characterized in that**:
at least one of said cover (102), said fixation element (104), and said elastic tube (106) is adapted to deform for regulating a pressure (402) applied by said fixation element (104) on said abdominal wall due to said tensile force (406); and
the pressure (402) applied by said fixation element (104) due to said tensile force (406) decreases to less than 10 mmHg when a colonic pressure is equal to or greater than approximately 10 mmHg.

2. An ostomy port (100) according to claim 1, comprising a cap (1114) for sealing an opening to said elastic tube (106).

3. An ostomy port (100) according to claim 2, wherein said cap (1114) includes a shell (1111) for accommodating a waste content collection bag (1112).

4. An ostomy port (100) according to claim 1 wherein said elastic tube (106) includes a material of durometer ranging from 5 to 50 shore A for providing said axial elasticity.

5. An ostomy port (100) according to claim 1 wherein a portion of said elastic tube (106) is adapted to bend for allowing relative motion between said cover (102) and said fixation element (104).

6. An ostomy port (100) according to claim 1 where said elastic tube (106) is collapsible for allowing peristaltic propelling of waste content.

7. An ostomy port (100) according to claim 1 wherein said fixation element (104) deforms in an axial direction when pressed against the abdominal wall.

8. An ostomy port (100) according to claim 7 wherein a pressure applied by said fixation element on said abdominal wall ranges from 1 to 200 mmHg.

9. An ostomy port (100) according to claim 1 wherein said cover (102) is adapted to elastically press against the abdominal wall.

10. An ostomy port (600A, 600B) according to claim 1 wherein said cover (602A, 602B) is deformable by 5 mm in an axial direction.

11. An ostomy port (100) according to claim 1 wherein said cover (102) is substantially mushroom-shaped.

12. An ostomy port (1300, 1400) according to claim 1 wherein said cover (1302, 1402) includes a shape which is adjustable to geometrical variations in abdominal wall thickness.

13. An ostomy port (100) according to any of claims 1 - 12 wherein said elastic tube (106) is pliable enough to collapse.

14. An ostomy port (100) according to any of claims 1 - 12 wherein said deformation is an elastic deformation.

15. A method for preventing leakage from a stoma on an abdominal wall comprising:
applying tension to a cover (102) and a fixation element (104) on opposing sides of the stoma with a collapsible pliable tube (106);
wherein at least one of said recover (102), said fixation element (104) and said tube (106) is adapted to elastically deform for regulating a pressure (402) applied by said fixation element (104) on said abdominal wall due to said tensile force (406); and
**characterized by** the pressure (402) applied by said fixation element (104) due to said tensile force (406) decreasing to less than 10 mmHg when a colonic pressure is equal to or greater than approximately 10 mmHg.

## Patentansprüche

1. Ostomieanschluss (100) zum Leiten von Abfallinhalt von einem intestinalen Abschnitt in einem abdominalen Hohlraum durch ein Stoma in einer abdominalen Wand, umfassend:
eine Abdeckung (102) zur Abdeckung des Stomas;
ein Befestigungselement (104) zur Verankerung des Anschlusses an einer viszeralen Seite der abdominalen Wand;
eine biegsame, axial plastische Röhre (106), die die Abdeckung (102) und das Befestigungselement (104) miteinander verbindet, und die so bemessen ist,
dass sei eine Zugkraft (406) auf die Abdeckung (102) und das Befestigungselement (104) ausübt;
**dadurch gekennzeichnet,**
**dass** wenigstens die Abdeckung (102), das Befestigungselement (104) oder die elastische Röhre (106) geeignet sind, sich zur Regulierung eines Drucks (402), der von dem Befestigungselement (104) auf die abdominale Wand aufgrund der Zugkraft (406) ausgeübt wird, zu verformen; und
**dass** der Druck (402), der vom Befestigungselement (104) aufgrund der Zugkraft (406) ausgeübt wird, auf weniger als 10 mm Hg fällt, wenn der Druck im Darm gleich oder größer als ungefähr 10 mm Hg beträgt.

2. Ostomieanschluss (100) nach Anspruch 1, umfassend eine Kappe (1114) zum Abdichten einer Öffnung zur elastischen Röhre (106).

3. Ostomieanschluss (100) nach Anspruch 2, wobei die Kappe (1114) ein Gehäuse (1111) zur Aufnahme eines Abfallsammelbeutels (1112) umfasst.

4. Ostomieanschluss (100) nach Anspruch 1, wobei die elastische Röhre (106) ein Material der Härte zwischen 5 und 50 Shore A umfasst, um die axiale Elastizität zu liefern.

5. Ostomieanschluss (100) nach Anspruch 1, wobei ein Abschnitt der elastischen Röhre (106) sich krümmen kann, um eine Relativbewegung zwischen der Abdeckung (102) und dem Befestigungselement (104) zu ermöglichen.

6. Ostomieanschluss (100) nach Anspruch 1, wobei die elastische Röhre (106) zusammenziehbar ist, um eine peristaltische Förderung des Abfallinhalts zu ermöglichen.

7. Ostomieanschluss (100) nach Anspruch 1, wobei das Befestigungselement (104) sich in axialer Richtung verformt, wenn es gegen die abdominale Wand gepresst wird.

8. Ostomieanschluss (100) nach Anspruch 7, wobei ein vom Befestigungselement auf die abdominale Wand ausgeübter Druck im Bereich von 1 bis 200 mmHg liegt.

9. Ostomieanschluss (100) nach Anspruch 1, wobei die Abdeckung (102) derart gestaltet ist, dass sie elastisch gegen die abdominale Wand drückt.

10. Ostomieanschluss (600A, 600B) nach Anspruch 1, wobei die Abdeckung (602A, 602B) um 5mm in axialer Richtung deformierbar ist.

11. Ostomieanschluss (100) nach Anspruch 1, wobei die Abdeckung (102) im Wesentlichen pilzförmig ist.

12. Ostomieanschluss (1300, 1400) nach Anspruch 1, wobei die Abdeckung (1302, 1402) eine Form umfasst, die anpassbar an geometrische Unterschiede in der Dicke der abdominalen Wand ist.

13. Ostomieanschluss (100) nach einem der Ansprüche 1 bis 12, wobei die elastische Röhre (106) genügend nachgiebig ist, um zusammenzufallen.

14. Ostomieanschluss nach einem der Ansprüche 1 bis 12, wobei die Deformation eine elastische Deformation ist.

15. Verfahren zur Verhinderung von Leckagen eines Stomas in einer abdominalen Wand, umfassend:
Ausüben eines Zugs auf eine Abdeckung (102) und ein Befestigungselement (104) auf gegenüberliegenden Seiten des Stomas mit einer zusammendrückbaren, biegsamen Röhre (106);
wobei zumindest die Abdeckung (102), das Befestigungselement (104) oder die Röhre (106) elastisch deformierbar ist, um einen Druck (402) zu regeln, der von dem Befestigungselement (104) auf die abdominale Wand aufgrund der Zugkraft (406) ausgeübt wird;
**gekennzeichnet dadurch, dass** der Druck (402), der durch das Befestigungselement (104) aufgrund der Zugkraft (406) ausgeübt wird, auf weniger als 10 mmHg fällt, wenn der Darmdruck gleich oder größer als ungefähr 10 mmHg beträgt.

## Revendications

1. Orifice de Stomie (100) pour conduire des déchets depuis une partie intestinale dans une cavité abdominale à travers un stoma dans une paroi abdominale comprenant :
un couvercle (102) pour couvrir le stoma ;
un élément de fixation (104) pour fixer l'orifice à un coté viscéral de la paroi abdominale ;
un tube (106) pliable et axialement élastique raccordant ledit couvercle (102) et ledit élément de fixation (104) et dimensionné pour appliquer une force de traction (406) audit couvercle (102) et audit élément de fixation (104) ;
**caractérisé en ce**
**qu'**au moins ledit couvercle (102) ou ledit élément de fixation (104) ou ledit tube élastique (106) sont aptes à se déformer pour régler une pression (402) appliquée par ledit élément de fixation (104) à ladite paroi abdominale à la suite de la force de traction (406); et
**que** la pression (402) appliquée par ledit élément de fixation (104) à la suite de la force de traction (406) diminue à moins de 10 mmHg si la pression colonique est égale ou supérieure à environ 10 mmHg.

2. Orifice de Stomie (100) selon la revendication 1, comprenant un bouchon (1114) pour obturer une ouverture vers le tube élastique (106).

3. Orifice de Stomie (100) selon la revendication 2, où ledit bouchon (1114) comprend un boitier (1111) pour recevoir une poche de collection de déchets (1112).

4. Orifice de Stomie (100) selon la revendication 1, où ledit tube élastique (106) comprend un matériau d'une dureté entre 5 et 50 Shore A pour fournir ladite élasticité axiale.

5. Orifice de Stomie (100) selon la revendication 1, où une section dudit tube élastique (106) est adaptée à se plier pour permettre un mouvement relatif entre ledit couvercle (102) et ledit élément de fixation (104).

6. Orifice de Stomie (100) selon la revendication 1, où ledit tube élastique (106) est compressible pour permettre l'avancement péristaltique de déchets.

7. Orifice de Stomie (100) selon la revendication 1, où l'élément de fixation (104) se déforme dans une direction axiale s'il est pressé contre la paroi abdominale.

8. Orifice de Stomie (100) selon la revendication 7, où une pression appliquée par ledit élément de fixation à ladite paroi abdominale est dans la plage de 1 à 200 mmHg.

9. Orifice de Stomie (100) selon la revendication 1, où ledit couvercle (102) est adapté à presser élastiquement sur la paroi abdominale.

10. Orifice de Stomie (600A, 600B) selon la revendication 1, où ledit couvercle (602A, 602B) est déformable de 5mm dans la direction axiale.

11. Orifice de Stomie (100) selon la revendication 1, où ledit couvercle (102) a essentiellement la forme d'un champignon.

12. Orifice de Stomie (1300, 1400) selon la revendication 1, où ledit couvercle (1302, 1402) comprend une forme qui est ajustable à des variations géométriques de l'épaisseur de la paroi abdominale.

13. Orifice de Stomie (100) selon l'une quelconque des revendications 1 à 12, où ledit tube élastique (106) est pliable assez pour s'effondrer.

14. Orifice de Stomie (100) selon l'une quelconque des revendications 1 à 12, où ladite déformation est une déformation élastique.

15. Procédé pour éviter une fuite d'un stoma dans une paroi abdominale comprenant :
l'application d'une traction à un couvercle (102) et à un élément de fixation (104) sur des cotés opposés d'un stoma avec un tube pliable et déformable (106) ;
où au moins ledit couvercle (102), ledit élément de fixation (104) ou ledit tube (106) sont adaptés à se déformer de manière élastique pour régler la pression (402) appliquée par l'élément de fixation (104) à ladite paroi abdominale suite aux forces de traction (406) ; et
**caractérisé en ce que** la pression (402) appliquée par ledit élément de fixation (104) à la suite de la force de traction (406) diminue à moins de 10 mmHg si la pression colonique est égale ou supérieure à environ 10 mmHg.
